# FASCICULE DE BREVET EUROPEEN

(11) **EP 2 604 227 B1**
(45) Date de publication et mention de la délivrance du brevet: **25.03.2015**
(21) Numéro de dépôt: 12193982.1
(22) Date de dépôt: 23.11.2012
(51) Int. Cl.: A61F 2/40, A61F 2/36

(54) **Embase d'ancrage osseux et ensemble d'éléments incluant cette embase**
Knochenverankerungsplatte und Komplex von Elementen, der diese Verankerungsplatte umfasst
Bone anchoring base and set of elements including said base

(30) Priorité: 15.12.2011 FR 1161693
(43) Date de publication de la demande: 19.06.2013
(73) Titulaire: FX Solutions, 01000 Bourg en Bresse (FR)
(72) Inventeur: Lascar, Tristan, 06320 Cap D'Ail (FR); Martin, Jean-Jacques, 01000 Bourg en Bresse (FR); Obert, Laurent, 25320 Vorges les Pins (FR)
(74) Mandataire: Jeannet, Olivier

(56) Documents cités:
- WO-A1-02/17822
- WO-A1-03/051238
- DE-U1- 20 104 312
- US-A1- 2006 009 852

## Description

La présente invention concerne une embase d'ancrage permettant l'ancrage à un os d'une pièce articulaire d'une prothèse d'articulation, et un ensemble d'éléments permettant la constitution d'une prothèse d'articulation, incluant cette embase d'ancrage.

Cet os peut notamment être un humérus ou un fémur, et ladite pièce articulaire peut donc être la pièce formant la surface d'articulation humérale d'une prothèse d'articulation de l'épaule, ou la pièce formant la surface d'articulation fémorale d'une prothèse d'articulation de la hanche, ou encore une pièce de resurfaçage, c'est-à-dire de reconstitution au moins partielle de la surface articulaire native.

Il est bien connu de réaliser l'ancrage d'une pièce articulaire d'une prothèse d'articulation au moyen d'une tige médullaire effilée destinée à être insérée dans le canal médullaire de l'os, jusqu'au niveau de la diaphyse.

Une telle tige médullaire peut cependant être délicate à implanter lorsque l'os n'est pas de bonne qualité, particulièrement en cas d'ostéoporose sur un patient âgé. Il en est de même en cas de reprise d'une prothèse antérieurement implantée, cette implantation antérieure ayant déjà conduit à une ablation d'os plus ou moins importante. Dans un cas comme dans l'autre, l'immobilisation de la pièce articulaire par rapport à l'os peut ne pas être parfaite et ne pas parfaitement résister aux contraintes répétées subies par la prothèse au cours du temps.

De plus, lorsque l'os est de bonne qualité ou que le patient est relativement jeune, par exemple en cas d'une arthrose débutante, il est regrettable d'avoir à procéder au sacrifice osseux important que nécessite la mise en place d'une tige médullaire.

Il est également connu, particulièrement sur des prothèses d'épaule, des embases d'ancrage vissables, qui ont pour inconvénient de présenter un risque notable de dévissage.

La demande de brevet N° WO 00/62718 décrit une embase d'ancrage comprenant une paroi de base solidaire, sur un côté, d'une paroi circulaire perforée d'ancrage, et, du côté opposé, d'un plot de montage d'une tête d'articulation prothétique. Cette embase limite le sacrifice osseux à opérer mais peut présenter des problèmes de pérennité de tenue à l'os.

Les documents N° WO 02/17822 A1, US 2006/009852 A1, DE 201 04 312 U1 et WO 03/051238 A1 divulguent divers matériels selon l'art antérieur. En particulier, le document N° WO 02/17822 A1 divulgue une embase d'ancrage telle que définie dans le préambule de la revendication 1 annexée.

La présente invention vise à remédier à l'ensemble des inconvénients précités.

L'embase d'ancrage concernée comprend, de manière connue en soi, une paroi de base, des moyens de liaison à une pièce articulaire et des moyens d'ancrage osseux, reliés à la paroi de base.

Selon l'invention, lesdits moyens d'ancrage comprennent des portions de paroi séparées les unes des autres et situées à proximité du bord périphérique de ladite paroi de base, chacune de ces portions de paroi présentant un bord de base relié à ladite paroi de base, un bord d'extrémité libre opposé à ce bord de base, et deux bords latéraux s'étendant entre ce bord de base et ce bord d'extrémité libre ; au moins une desdites portions de paroi est disposée sur ladite paroi de base de telle sorte qu'un bord latéral de cette portion de paroi se trouve à une distance du centre de la paroi de base qui est différente de la distance à laquelle se trouve l'autre bord latéral de cette même portion de paroi par rapport à ce centre.

Ainsi, ladite portion de paroi est orientée différemment des portions de paroi adjacentes ; après l'implantation de l'embase selon l'invention, consistant à insérer lesdites portions de paroi dans l'os spongieux de l'os à appareiller par impaction, et une fois que s'est opérée la croissance des cellules osseuses autour desdites portions de paroi et que l'os s'est consolidé, l'embase selon l'invention a une stabilité d'ancrage importante, particulièrement en pivotement selon l'axe de l'impaction. Cette stabilité anti-rotatoire résulte de l'orientation différente de ladite portion de paroi et des surfaces importantes d'appui dans l'os que forment les bords latéraux desdites portions de paroi.

Grâce à cette stabilité importante, l'ancrage de l'embase selon l'invention est à même de parfaitement résister aux efforts répétés exercés sur cette embase par les mouvements articulaires.

Pour l'implantation de l'embase d'ancrage selon l'invention, l'os est coupé de manière à aménager une surface métaphysaire réséquée puis une rainure grossièrement à la forme des différentes portions de paroi est aménagée dans l'os à partir de cette surface. Cette rainure peut avoir une largeur correspondant plus ou moins à l'épaisseur des portions de paroi, voire légèrement inférieure à cette épaisseur, afin de permettre une insertion forcée des portions de paroi dans cette rainure, par impaction.

De préférence, lesdites portions de paroi sont régulièrement réparties sur l'ensemble du pourtour de ladite paroi de base et chaque portion de paroi est disposée sur cette paroi de base de la manière précitée, c'est-à-dire de manière telle qu'un bord latéral d'une portion de paroi se trouve à une distance du centre de la paroi de base qui est différente de la distance à laquelle se trouve l'autre bord latéral de cette même portion de paroi par rapport à ce centre.

Une stabilité anti-rotatoire particulièrement importante est ainsi obtenue.

De préférence, au moins un bord latéral est dentelé ou crénelé.

Ce bord latéral présente ainsi des encoches permettant, après lesdites croissance et consolidation osseuses, une résistance augmentée de l'ancrage de l'embase en arrachement selon l'axe d'insertion desdites portions de paroi dans l'os.

De préférence, les bords latéraux de chaque portion de paroi sont dentelés ou crénelés, pour une résistance à l'arrachement encore renforcée.

De préférence, le bord d'extrémité libre de chaque portion de paroi a une forme pointue, conférant à ce bord d'extrémité libre une certaine capacité à être auto-perforateur de l'os spongieux lors de l'insertion desdites portions de paroi dans cet os spongieux.

De préférence, des reliefs d'ancrage osseux sont aménagés sur la face interne et/ou la face externe d'au moins une portion de paroi. Ces reliefs d'ancrage osseux sont de préférence aménagés sur toutes lesdites portions de paroi.

Ces reliefs d'ancrage osseux contribuent à assurer le parfait ancrage de l'embase selon l'invention.

Lesdits moyens de liaison à une pièce articulaire peuvent notamment être sous la forme d'un plot solidaire de l'embase ou de ladite pièce articulaire et d'une cavité de réception de ce plot avec coincement, aménagée dans ladite pièce articulaire ou dans l'embase. Ces mêmes moyens de liaison peuvent également être sous la forme de deux cavités, une aménagée dans l'embase et l'autre aménagée dans ladite pièce articulaire, et d'une pièce de liaison apte à être engagée et retenue dans ces deux cavités.

Selon une forme de réalisation préférée de l'invention, dans ce deuxième cas, l'embase comprend une paroi tubulaire solidaire de ladite paroi de base, faisant saillie de cette paroi de base sur le même côté de celle-ci que lesdites portions de paroi, cette paroi tubulaire délimitant une cavité de réception avec coincement d'une pièce de liaison à ladite pièce articulaire.

Cette paroi tubulaire contribue à l'ancrage de l'embase dans l'os spongieux.

Ladite paroi tubulaire comprend avantageusement des reliefs d'ancrage osseux sur sa face externe, notamment sous forme de collerettes.

L'ensemble d'éléments que concerne également l'invention comprend :
- au moins une embase d'ancrage telle que précitée ;
- au moins une pièce articulaire apte à être montée sur cette embase,
- au moins une pièce de resurfaçage, c'est-à-dire de reconstitution au moins partielle de la surface articulaire native ;
- des moyens de liaison permettant l'assemblage de l'une ou l'autre de ces pièces articulaires et de resurfaçage sur chaque embase d'ancrage.

L'ensemble d'éléments selon l'invention comprend de préférence une pluralité d'embases d'ancrage de différentes tailles et dimensions.

Lorsque la prothèse à constituer est une prothèse d'épaule, l'ensemble d'éléments comprend en outre au moins une pièce articulaire à surface d'articulation convexe pour la constitution d'une prothèse dite "anatomique" et au moins une pièce articulaire à surface d'articulation concave pour la constitution d'une prothèse dite "inversée".

Les moyens de liaison comprennent de préférence une cavité conique sur chaque embase d'ancrage et une cavité conique sur au moins une pièce articulaire et des pièces de liaison à double portions d'assemblage coniques, une de ces portions d'assemblage coniques étant apte à être engagée et retenue dans la cavité conique d'une embase d'ancrage et l'autre de ces portions d'assemblage coniques étant apte à être engagée et retenue dans la cavité conique de la pièce articulaire ; l'ensemble d'éléments selon l'invention comprend alors de préférence :
- au moins une pièce de liaison droite ;
- au moins une pièce de liaison angulée, c'est-à-dire dont l'axe d'une portion d'assemblage forme un angle différent de 180° avec l'axe de l'autre portion d'assemblage ;
- au moins une pièce de liaison décalée, c'est-à-dire dont l'axe d'une portion d'assemblage est décalé, au niveau de l'interface de liaison de cette portion avec l'autre portion, par rapport à l'axe de l'autre portion.

L'invention sera bien comprise, et d'autres caractéristiques et avantages de celle-ci apparaîtront, en référence au dessin schématique annexé, représentant, à titre d'exemples non limitatifs, plusieurs formes de réalisation possibles de l'embase d'ancrage et de l'ensemble d'éléments concernés.

La figure 1 est une vue de côté de différents éléments constituant l'ensemble d'éléments concerné, dont une embase d'ancrage selon une première forme de réalisation, ledit ensemble d'éléments étant destiné à constituer une prothèse d'épaule ; la figure 1 montre également la partie supérieure d'un humérus, dont la métaphyse a été réséquée pour recevoir ladite embase d'ancrage ;
la figure 2 est une vue de l'embase d'ancrage en perspective ;
la figure 3 en est une vue de dessous ;
la figure 4 montre la partie supérieure d'un humérus équipé d'une prothèse d'articulation constituée à partir d'éléments montrés sur la figure 1 ;
les figures 5 à 7 sont des vues de côté de plusieurs autres éléments que comprend ledit ensemble d'éléments, après mise en place sur un humérus ;
la figure 8 est une vue de côté de divers éléments permettant la constitution d'une prothèse d'épaule, dont une embase d'ancrage selon une deuxième forme de réalisation ; la figure 8 montre également la partie supérieure d'un humérus, dont la métaphyse a été réséquée pour recevoir ladite embase d'ancrage ;
la figure 9 est une vue de l'embase d'ancrage en perspective ;
la figure 10 en est une vue de dessous ;
la figure 11 est une vue similaire à la figure 9 de l'embase d'ancrage selon une variante de réalisation ;
la figure 12 est une vue similaire à la figure 2 de l'embase d'ancrage selon une autre variante de réalisation ; et
la figure 13 est une vue similaire à la figure 9 de l'embase d'ancrage selon cette dernière variante de réalisation.

Par simplification, les parties ou éléments d'une forme de réalisation qui se retrouvent de manière identique ou similaire sur une autre forme de réalisation seront identifiés par les mêmes références numériques et ne seront pas à nouveau décrits.

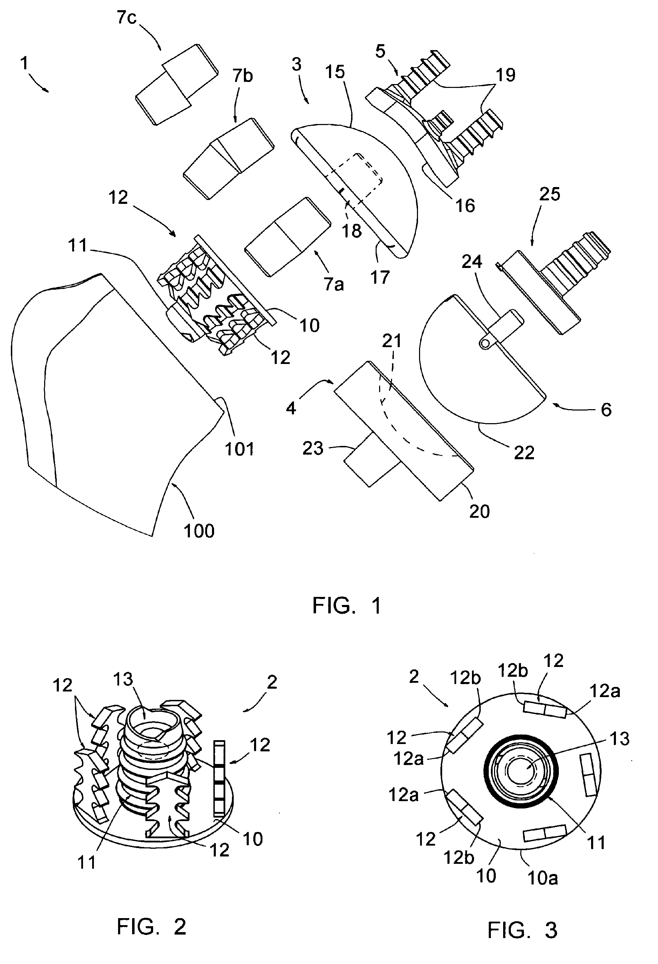

La figure 1 représente un ensemble 1 de différents éléments permettant la constitution d'une prothèse d'articulation de l'épaule, à savoir :
- une embase d'ancrage 2 ;
- des pièces articulaires 3, 4 destinées à être montées sur l'humérus 100, dont une pièce 3 à surface d'articulation convexe et une pièce 4 à surface d'articulation concave ;
- des pièces articulaires 5, 6 destinées à être montées sur l'omoplate, dont une pièce 5 à surface d'articulation concave, destinée à être conjuguée à la pièce 3 et une pièce 6 à surface d'articulation convexe, destinée à être conjuguée à la pièce 4 ; et
- des pièces de liaison 7a, 7b, 7c biconiques.

En référence aux figures 1 à 3, il apparaît que l'embase d'ancrage 2 comprend une paroi de base 10, une paroi tubulaire centrale 11 et des portions de paroi périphériques 12 d'ancrage osseux. L'ensemble est réalisé en une même pièce de métal biocompatible, par exemple en titane.

La paroi de base 10 est plane et de forme circulaire.

La paroi tubulaire centrale 11 est solidaire de la paroi de base 10 et fait saillie de cette dernière sur le même côté de celle-ci que les portions de paroi 12. Elle délimite une cavité légèrement conique 13 (environ 5 à 10° de pente) de réception avec coincement d'une portion conique correspondante de l'une ou l'autre des pièces de liaison 7a à 7c.

La paroi tubulaire 11 comprend en outre des collerettes étagées sur sa face externe, formant des reliefs d'ancrage osseux.

Les portions de paroi périphériques 12 d'ancrage osseux sont séparées les unes des autres et sont au nombre de cinq dans l'exemple montré sur les figures 1 à 3. Chacune d'elles présente un bord de base par lequel elle est solidaire de la paroi de base 10, un bord d'extrémité libre opposé à ce bord de base, et deux bords latéraux s'étendant entre ce bord de base et ce bord d'extrémité libre, et fait saillie de la paroi de base 10 selon une direction sensiblement perpendiculaire au plan de cette paroi 10.

Les bords d'extrémité libre des différentes portions de paroi 12 sont pointus, conférant à ces portions de paroi 12 une certaine capacité d'auto-perforation de l'os spongieux d'un humérus 100, et les bords latéraux des différentes portions de paroi 12 sont dentelés, c'est-à-dire présentent des formes ondulées définissant une succession de saillies pointues et d'encoches

Les portions de paroi 12 sont, dans l'exemple de réalisation montré sur les figures 1 à 3, disposées sur l'ensemble du pourtour de la paroi de base 10, en étant régulièrement réparties sur ce pourtour.

Comme cela est particulièrement visible sur la figure 3, chaque portion de paroi 12 n'est pas disposée le long du bord périphérique 10a de la paroi de base 10 mais est disposée en oblique par rapport à ce bord, de telle sorte que l'un de ses bords latéraux 12a se situe à proximité de ce bord périphérique 10a et se trouve à une première distance du centre de la paroi de base 10, tandis que l'autre bord latéral 10b se situe plus en retrait dudit bord périphérique 10a et se trouve à une deuxième distance du centre de la paroi de base 10, cette deuxième distance étant inférieure à ladite première distance. Chaque paroi 12 est substantiellement plane, et ce plan forme un angle de l'ordre de 10° avec une tangente à la paroi de base 10 au point du bord périphérique 10a dont le bord latéral 12a se trouve proche.

En outre, il apparaît sur la figure 3 que l'orientation oblique d'une portion de paroi 12 est alternée avec l'orientation oblique de la portion de paroi 12 consécutive, c'est-à-dire, ainsi que cela est décrit ci-après :
- les deux portions de paroi 12 situées sur la gauche de cette figure 3 ont leurs bords latéraux 12a disposés en regard l'un de l'autre, et, par conséquent, leurs deux bords latéraux 12b situés à l'opposé l'un de l'autre ;
- dans les deux paires de portions de paroi 12 consécutives à cette première paire, formées chacune par l'une des portions de paroi 12 de cette première paire et par la portion de paroi 12 adjacente (il s'agit donc des paires de portions de paroi 12 situées sur le côté supérieur et sur le côté inférieur de la figure 3), les bords latéraux 12b des deux portions de paroi 12 sont disposés en regard l'un de l'autre, et, par conséquent, les deux bords latéraux 12a sont situés à l'opposé l'un de l'autre ;
- ces orientations obliques alternées se retrouvent sur l'ensemble du pourtour de l'embase 2, à l'exception, dans l'exemple représenté, d'une dernière paire de portions de paroi 12 (il s'agit, sur la figure 3, de celle située sur la droite et sur le côté supérieur), étant donné que l'embase 2 comprend un nombre impair (cinq) de portions de paroi 12 ; au sein de cette paire de portions de paroi 12, les deux portions de paroi 12 ont une même orientation.

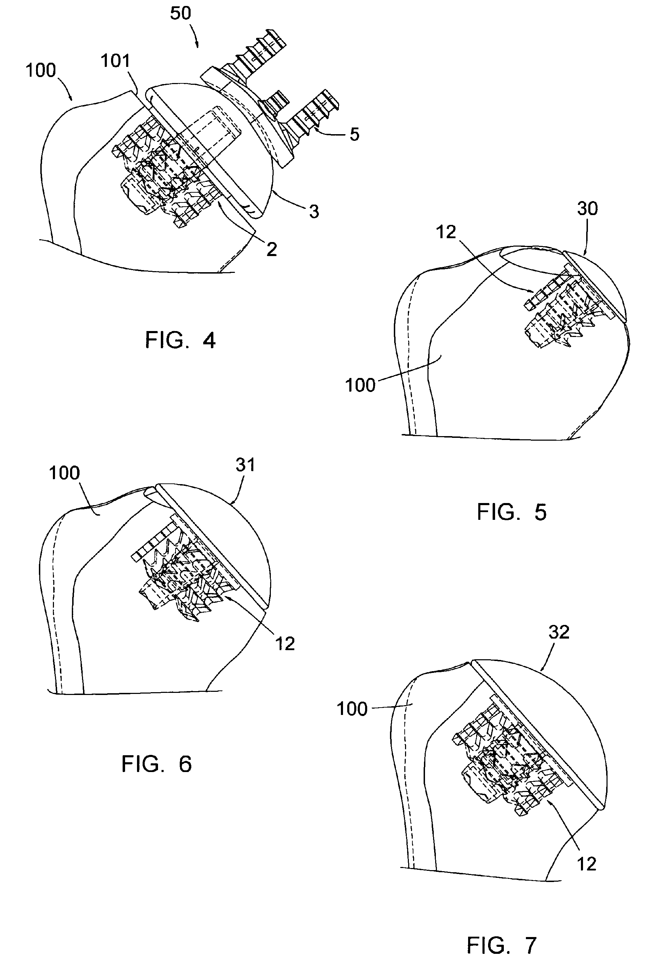

La pièce articulaire 3 présente une face convexe 15 formant une surface articulaire apte à coopérer avec une surface articulaire concave conjuguée 16 aménagée par la pièce articulaire 5, et une face plane 17 venant, en position de montage (cf. figure 4), à proximité immédiate de ladite paroi de base 10. Au centre de cette face plane 17 est aménagée une cavité conique 18 à faible pente apte à recevoir avec coincement une des deux portions en cône de l'une ou l'autre des pièces de liaison 7a, 7b, 7c.

La pièce articulaire 5 comprend, outre le plateau aménageant la surface articulaire 16, des plots 19 d'insertion dans l'os de l'omoplate.

La pièce articulaire 4 présente un plateau 20 définissant une face concave 21 qui forme une surface articulaire apte à coopérer avec une surface articulaire convexe conjuguée 22 aménagée par la pièce articulaire 6, et un plot conique à faible pente 23 apte à être engagé avec coincement dans la cavité 13.

La pièce articulaire 6 comprend, outre la paroi aménageant la surface articulaire 21, un plot 24 apte à être reçu dans une cavité correspondante aménagée dans une embase 25 d'ancrage à l'omoplate.

La pièce de liaison 7a est droite, c'est-à-dire que ses deux portions coniques opposées ont les axes sont confondus.

La pièce de liaison 7b est angulée, c'est-à-dire que ses deux portions coniques opposées ont des axes formant entre eux un angle différent de 180°.

La pièce de liaison 7c est décalée, c'est-à-dire que ses deux portions coniques opposées ont des axes décalés l'un par rapport à l'autre au niveau de l'interface de liaison de ces portions l'une à l'autre.

En pratique, l'os 100 est coupé de manière à aménager une surface métaphysaire réséquée 101, puis une rainure circulaire, au diamètre défini par les axes médians longitudinaux des différentes portions de paroi 12, est aménagée dans l'os à partir de cette surface 101, de même qu'une cavité de réception avec coincement du plot formé par ladite paroi tubulaire centrale 11. La rainure a une largeur correspondant plus ou moins à l'épaisseur des portions de paroi 12, voire légèrement inférieure à cette épaisseur, afin de permettre une insertion forcée des portions de paroi 12 dans cette rainure, par impaction.

L'embase 2 est ensuite impactée dans cette rainure jusqu'à venue de la face de la paroi de base 10 comportant les portions de paroi 12 au contact de la face 101. La pièce de liaison 7a, 7b ou 7c adaptée à la morphologie du patient est ensuite mise en place sur l'embase 2 par insertion et coincement dans la cavité 13 puis la pièce articulaire 3 est mise en place par insertion de la pièce de liaison 7a, 7b ou 7c dans la cavité 18 de cette pièce. La pièce articulaire 5 est par ailleurs mise en place sur l'omoplate et les surfaces articulaires de ces pièces 3, 5 sont amenées au contact l'une de l'autre pour former ainsi une prothèse 50 d'articulation de l'épaule.

Une fois que s'est opérée la croissance des cellules osseuses autour des portions de paroi 12 et que l'os s'est consolidé, l'embase 2 a une stabilité d'ancrage importante, tant en pivotement selon l'axe de l'impaction, compte tenu des orientations obliques alternées précitées des portions de paroi 12, qu'en arrachement selon ce même axe d'insertion, compte tenu des dentelures des bords latéraux de ces mêmes portions de paroi 12.

Grâce à cette stabilité importante, l'ancrage de l'embase 2 est à même de parfaitement résister aux efforts répétés exercés sur cette embase par les mouvements articulaires.

En lieu et place des pièces 3 et 5, les pièces 4, 6 et 25 peuvent être utilisées s'il s'avère indiqué de constituer une prothèse inversée.

Les figures 5 et 6 montrent que l'ensemble d'éléments peut en outre inclure des embases 2 de plus petits diamètres, comprenant un nombre différent de portions de paroi 12, et/ou des portions de paroi 12 non régulièrement réparties sur le pourtour de la paroi de base 10, et comprenant des parois centrales tubulaires 11 de plus petits diamètres. Notamment, dans l'exemple de réalisation montré sur la figure 5, l'embase 2 comprend deux portions de paroi 12, et, dans l'exemple de réalisation montré sur la figure 6, ou quatre portions de paroi 12.

Ces embases 2 de plus petits diamètres sont aptes à recevoir des pièces articulaires 30, 31 de resurfaçage, c'est-à-dire de reconstitution au moins partielle de la surface articulaire native, de différents diamètres et épaisseurs.

La figure 7 montre qu'une embase 2 telle que décrite en référence aux figures 1 à 3 peut également recevoir une pièce articulaire 32 de resurfaçage complet de l'humérus proximal, monté sur elle de la même manière que précitée.

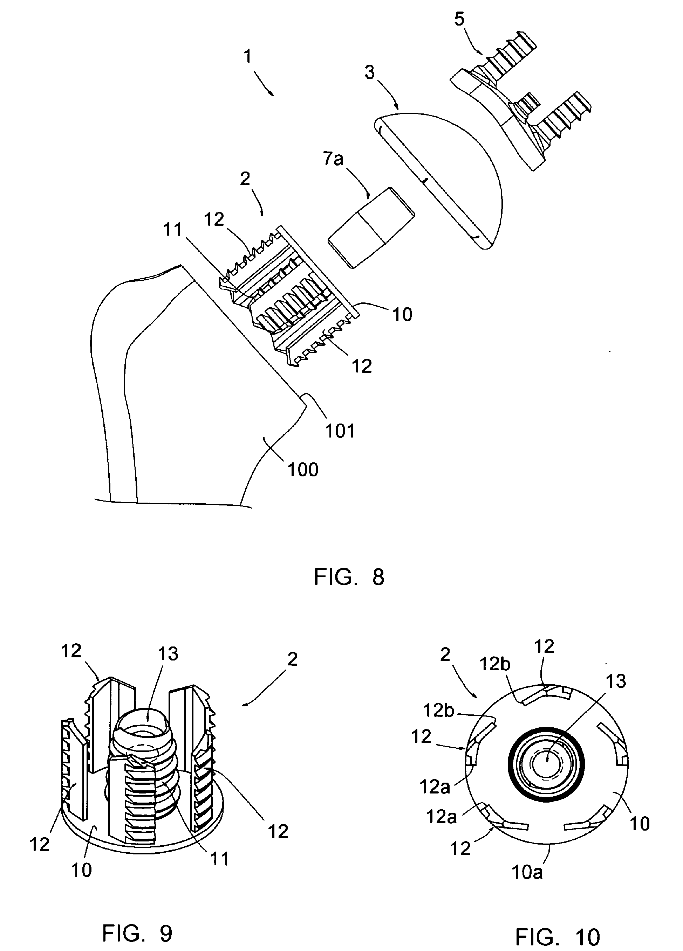

La figure 8 montre une embase 2 selon une deuxième forme de réalisation, et une pièce de liaison 7a, une pièce articulaire 3 et une pièce articulaire 5 identiques à celles décrites précédemment.

Selon cette deuxième forme de réalisation, ainsi que cela apparaît sur les figures 9 et 10, les portions de paroi 12 ne sont pas planes et chacune d'elles présente deux parties longitudinales distinctes : une partie d'épaisseur augmentée, située à proximité du bord périphérique 10a et pourvue de reliefs d'ancrage étagés, et une partie moins épaisse, sensiblement plane et lisse, située en retrait de ce bord périphérique 10a et orientée obliquement de la manière précitée.

L'embase 2 selon cette deuxième forme de réalisation s'utilise exactement de la même manière que l'embase 2 selon la première forme de réalisation décrite précédemment.

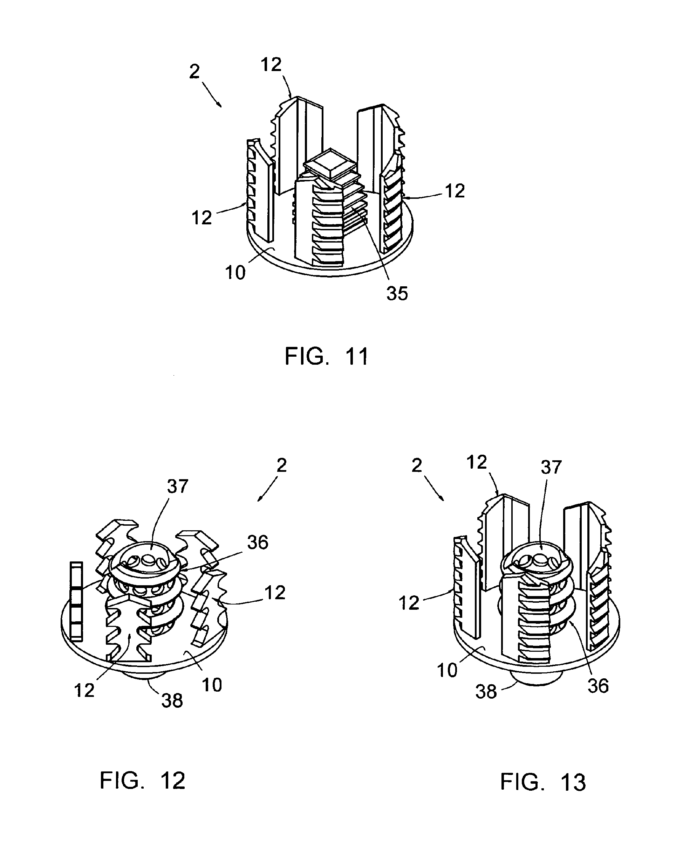

La figure 11 montre que l'embase 2 peut comprendre un plot central pyramidal 35 pourvu de collerettes étagées d'ancrage à l'os. Ce plot 35 peut former la cavité 13 ou peut être plein, auquel cas l'embase 2 comprend, sur sa face opposée à celle comportant les portions de parois 12, un plot conique de montage de la pièce articulaire 3, 4 ou 32. Sur cette figure 11, c'est l'embase 2 comprenant des portions de parois 12 selon la deuxième forme de réalisation qui est représentée, mais il est bien clair que l'embase selon la première forme de réalisation pourrait être équipée du plot 35.

Les figures 12 et 13 montrent des embases 2 respectivement selon la première et la deuxième forme de réalisation, qui sont équipées d'un plot central 36 formant une cavité 37 de réception d'un greffon et dont la paroi est multi-perforée pour permettre la croissance des cellules osseuses entre l'os spongieux et ce greffon. Ce plot 36 est pourvu de collerettes étagées d'ancrage à l'os. Bien entendu, dans ce cas, l'embase 2 comprend, sur sa face opposée à celle comportant les portions de parois 12, un plot conique 38 de montage de la pièce articulaire 3, 4 ou 32.

Comme cela apparaît de ce qui précède, l'invention fournit une embase d'ancrage 2 et un ensemble 1 d'éléments permettant la constitution d'une prothèse d'articulation, présentant des avantages déterminants par rapport aux matériels homologues selon la technique antérieure, l'embase 2 selon l'invention ayant en particulier une stabilité d'ancrage importante, et pouvant être utilisée, dans de nombreuses indications, en remplacement d'une tige médullaire classique.

L'invention a été décrite ci-dessus en référence à des formes de réalisation fournies à titre d'exemple. Il va de soi qu'elle n'est pas limitée à ces formes de réalisation mais qu'elle s'étend à toutes les autres formes de réalisations couvertes par les revendications ci-annexées.

## Revendications

1. Embase (2) d'ancrage permettant l'ancrage à un os (100) d'une pièce articulaire (3, 4) d'une prothèse d'articulation, comprenant une paroi de base (10), des moyens (13) de liaison à ladite pièce articulaire (3, 4) et des moyens (12) d'ancrage osseux, reliés à ladite paroi de base (10) ;
**caractérisée en ce que** lesdits moyens d'ancrage comprennent des portions de paroi (12) séparées les unes des autres et situées à proximité du bord périphérique de ladite paroi de base (10), chacune de ces portions de paroi (12) présentant un bord de base relié à ladite paroi de base (10), un bord d'extrémité libre opposé à ce bord de base, et deux bords latéraux s'étendant entre ce bord de base et ce bord d'extrémité libre ; au moins une desdites portions de paroi (12) est disposée sur ladite paroi de base (10) de telle sorte qu'un bord latéral (12a, 12b) de cette portion de paroi (12) se trouve à une distance du centre de la paroi de base (10) qui est différente de la distance à laquelle se trouve l'autre bord latéral (12b, 12a) de cette même portion de paroi (12) par rapport à ce centre.

2. Embase (2) d'ancrage (1) selon la revendication 1, **caractérisée en ce que** lesdites portions de paroi (12) sont régulièrement réparties sur l'ensemble du pourtour de ladite paroi de base (10) et **en ce que** chaque portion de paroi (12) est disposée sur cette paroi de base (10) de la manière précitée, c'est-à-dire de manière telle qu'un bord latéral (12a, 12b) d'une portion de paroi (12) se trouve à une distance du centre de la paroi de base (10) qui est différente de la distance à laquelle se trouve l'autre bord latéral (12b, 12a) de cette même portion de paroi (12) par rapport à ce centre.

3. Embase (2) d'ancrage (1) selon la revendication 1 ou la revendication 2, **caractérisée en ce qu'**au moins un bord latéral est dentelé ou crénelé.

4. Embase (2) d'ancrage (1) selon la revendication 3, **caractérisée en ce que** les bords latéraux de chaque portion de paroi (12) sont dentelés ou crénelés.

5. Embase (2) d'ancrage (1) selon l'une des revendications 1 à 4, **caractérisée en ce que** le bord d'extrémité libre de chaque portion de paroi (12) a une forme pointue.

6. Embase (2) d'ancrage (1) selon l'une des revendications 1 à 5, **caractérisée en ce que** des reliefs d'ancrage osseux sont aménagés sur la face interne et/ou la face externe d'au moins une portion de paroi (12).

7. Embase (2) d'ancrage (1) selon l'une des revendications 1 à 6, **caractérisée en ce qu'**elle comprend une paroi tubulaire (11) solidaire de ladite paroi de base (10), faisant saillie de cette paroi de base (10) sur le même côté de celle-ci que lesdites portions de paroi (12), cette paroi tubulaire (11) délimitant une cavité (13) de réception avec coincement d'une pièce de liaison (7a à 7c) à ladite pièce articulaire (3, 4).

8. Embase (2) d'ancrage (1) selon la revendication 7, **caractérisée en ce que** ladite paroi tubulaire (11) comprend des reliefs d'ancrage osseux sur sa face externe, notamment sous forme de collerettes.

9. Embase (2) d'ancrage (1) selon l'une des revendications 1 à 6, **caractérisée en ce qu'**elle comprend un plot central pyramidal (35).

10. Embase (2) d'ancrage (1) selon l'une des revendications 1 à 6, **caractérisée en ce qu'**elle comprend un plot central (36) formant une cavité (37) de réception d'un greffon et dont la paroi est multi-perforée pour permettre la croissance des cellules osseuses entre l'os spongieux et ce greffon.

11. Ensemble (1) d'éléments permettant la constitution d'une prothèse d'articulation (50), caractérisé **en ce qu'il** comprend :
- au moins une embase d'ancrage (2) selon l'une des revendications 1 à 10 ;
- au moins une pièce articulaire (3, 4) apte à être montée sur cette embase (2),
- au moins une pièce de resurfaçage (30 à 32), c'est-à-dire de reconstitution au moins partielle de la surface articulaire native ;
- des moyens de liaison (7a à 7c, 13, 18) permettant l'assemblage de l'une ou l'autre de ces pièces articulaires (3, 4) et de resurfaçage (30 à 32) sur chaque embase d'ancrage (2).

12. Ensemble d'éléments selon la revendication 11, **caractérisé en ce qu'il** comprend une pluralité d'embases (2) d'ancrage de différentes tailles et dimensions.

13. Ensemble d'éléments selon la revendication 11 ou revendication 12, caractérisé **en ce qu'il** comprend au moins une pièce articulaire (3) à surface d'articulation convexe pour la constitution d'une prothèse d'épaule (50) dite "anatomique" et au moins une pièce articulaire (4) à surface d'articulation concave pour la constitution d'une prothèse d'épaule dite "inversée".

14. Ensemble d'éléments selon l'une des revendications 11 à 13, **caractérisé :**
- **en ce que** lesdits moyens de liaison comprennent une cavité conique (13) sur chaque embase (2) d'ancrage et une cavité conique sur au moins une pièce articulaire (3) et des pièces de liaison (7a à 7c) à double portions d'assemblage coniques, une de ces portions d'assemblage coniques étant apte à être engagée et retenue dans la cavité conique (13) d'une embase (2) d'ancrage et l'autre de ces portions d'assemblage coniques étant apte à être engagée et retenue dans la cavité conique (18) de la pièce articulaire (3) ; et
- **en ce que** l'ensemble (1) d'éléments comprend au moins une pièce de liaison droite (7a), au moins une pièce de liaison angulée (7b), c'est-à-dire dont l'axe d'une portion d'assemblage forme un angle différent de 180° avec l'axe de l'autre portion d'assemblage, et au moins une pièce de liaison décalée (7c), c'est-à-dire dont l'axe d'une portion d'assemblage est décalé, au niveau de l'interface de liaison de cette portion avec l'autre portion, par rapport à l'axe de l'autre portion.

## Patentansprüche

1. Basis (2) Anker zur Verankerung in einem Knochen (100) von einem Gelenkteil (3, 4) einer Gelenkprothese, umfassend eine Basiswand (10), Mittel (13) zur Verbindung mit der Gelenkteil (3, 4) und Mittel (12) der Knochenanker, die mit der Basiswand (10) verbunden sind;
**dadurch gekennzeichnet, dass** die Verankerungsmittel umfassen Wandabschnitte (12) voneinander getrennt sind und in der Nähe der Umfangskante der Basiswand (10), wobei jeder der Wandabschnitte (12) mit einer Basiskante, die mit der Basiswand (10), ein freies Ende Kante gegenüber der Basiskante und zwei Seitenkanten, die sich zwischen diesen Basiskante und diese freie Stirnkante; zumindest einer der Wandabschnitte (12) auf der Basiswand (10) angeordnet ist, so dass eine Seitenkante (12a, 12b) des Wandabschnitts (12) ist in einem Abstand von der Mitte der Basiswand (10) die sich von dem Abstand von der anderen Seitenkante (12b, 12a) der genannten Wandabschnitte (12) relativ zu der Mitte ist.

2. Basis (2) Anker (1) nach Anspruch 1, **dadurch gekennzeichnet, dass** die Wandabschnitte (12) regelmäßig über den gesamten Umfang der Basiswand (10) verteilt und dass jeder Wandabschnitt (12) an dieser Bodenwand (10) angeordnet ist, wie oben erwähnt, das heißt in einer solchen Weise zu sagen, dass eine Seitenkante (12a, 12b) des Wandabschnitts (12) ist in einem Abstand von der Mitte der Basiswand (10) die sich von dem Abstand von der anderen Seitenkante (12b, 12a) der genannten Wandabschnitte (12) relativ zu der Mitte ist.

3. Basis (2) Anker (1) nach Anspruch 1 oder Anspruch 2, **dadurch gekennzeichnet, dass** mindestens eine Seitenkante gezackt oder Zinnen ist.

4. Basis (2) Anker (1) nach Anspruch 3, **dadurch gekennzeichnet, dass** die Seitenkanten jedes Wandteils (12) gezackt oder Zinnen geformt sind.

5. Boden (2) Anker (1) nach einem der Ansprüche 1 bis 4, **dadurch gekennzeichnet, dass** die das freie Ende Kante jedes Wandabschnitte (12) eine spitz zulaufende Form hat.

6. Base (2) Anker (1) nach einem der Ansprüche 1 bis 5, **dadurch gekennzeichnet, dass** Knochenverankerungsreliefs an der Innenfläche und / oder auf der Außenfläche von mindestens einem Wandabschnitte (12) angeordnet sind.

7. Base (2) Anker (1) nach einem der Ansprüche 1 bis 6, **dadurch gekennzeichnet, dass** sie umfasst eine röhrenförmige Wand (11) integral mit der Basiswand (10), auf der gleichen Seite davon wie die Wandabschnitte (12) vorstehen, wobei diese rohrförmige Wand (11) die einen Hohlraum (13) zur Aufnahme mit Klemm ein Verbindungsstück (7a bis 7c) mit dem Gelenkteil (3, 4).

8. Basis (2) Anker (1) nach Anspruch 7, **dadurch gekennzeichnet, dass** die rohrförmige Wand (11) aufweist Knochenverankerungsreliefs an seiner Außenseite, insbesondere in Form von Ringen.

9. Basis (2) Anker (1) nach einem der Ansprüche 1 bis 6, **dadurch gekennzeichnet, dass** sie einen pyramidenförmigen Mittelstück (35) aufweist.

10. Basis (2) Anker (1) nach einem der Ansprüche 1 bis 6, **dadurch gekennzeichnet, dass** es umfasst eine Mittelstück (36) einen Hohlraum (37) aufweist, zur Aufnahme eines Transplantats, wobei die Wand ist mit mehreren perforierten, um das Wachstum von Knochenzellen , die zwischen der Spongiosa und dieses Transplantats ermöglichen.

11. Satz (1) von Elementen, die die Bildung einer Gelenkprothese (50), **dadurch gekennzeichnet, dass** sie umfasst:
- Mindestens einem Basis (2) nach einem der Ansprüche 1 bis 10;
- Mindestens ein Gelenkteil (3, 4) angepasst ist, um an der Basis (2) zu werden,
- Mindestens ein Stück Überlagerung (30 bis 32), das zumindest teilweise Wiederherstellung des natürlichen Gelenkfläche zu sagen;
- Verbindungsmittel (7a bis 7c, 13, 18) zum Zusammensetzen der einen oder der anderen dieser Gelenkteil (3, 4) und Resurfacing Teile (30 bis 32) auf jedem Ankerbasis (2).

12. Satz von Elementen nach Anspruch 11, **dadurch gekennzeichnet, dass** sie eine Vielzahl von Verankerungsbasen (2) mit unterschiedlicher Größe und Abmessungen aufweist.

13. Satz von Elementen nach Anspruch 11 oder Anspruch 12, **dadurch gekennzeichnet, dass** es umfasst mindestens ein Gelenkteil (3) Ausbilden einer konvexen Gelenkoberfläche für die Bildung eines sogenannten "anatomische" Schulterprothese (50), und mindestens ein Gelenkteil (4) eine konkave Gelenkoberfläche für die Bildung eines so genannten "Rückwärts" Schulterprothese.

14. Satz von Elementen nach Anspruch einem der Ansprüche 11 bis 13, **dadurch gekennzeichnet**:
- dass die Verbindungsmittel eine konische Vertiefung (13) auf jedem Verankerungsbasis (2) und einen konischen Hohlraum auf wenigstens einer Gelenkteil (3) und Verbindungsteile (7a bis 7c) verfügen über ein Doppel Konusverbindung Abschnitte, eine dieser konischen Verbindungsabschnitte in der Lage ist, in Eingriff gebracht und in dem konischen Hohlraum (13) einer Verankerungsbasis (2) das andere dieser konische Verbindungsabschnitte in der Lage in Eingriff gebracht werden kann und gehalten wird in dem konischen Hohlraum (18) des Gelenkteils (3); und
- Dass die Satz (1) von Elementen umfasst, mindestens einen geraden Anschlussteil (7a), mindestens einen abgewinkelten Verbindungsteil (7b), das heißt, die Achse einer Montageabschnitt bilden einen von 180° verschiedenen Winkel mit der Achse des anderen Verbindungsabschnitt und mindestens einem verschoben Verbindungselement (7c), das heißt, die Achse einer Montageabschnitt versetzt ist, an der Verbindung Grenzfläche dieses Abschnitts mit einem anderen Teil, relativ zu der Achse des anderen Teils.

## Claims

1. Anchoring base (2) for the anchoring of an articular part (3, 4) of a joint prosthesis in a bone (100), comprising a base wall (10), means (13) for connection to said articular part (3, 4) and bone anchoring means (12) connected to said base wall (10);
**characterized in that** said anchoring means comprise wall portions (12) separated from each other and located near the peripheral edge of said base wall (10), each of said wall portions (12) having a base edge connected to said base wall (10), a free end edge opposed to this base edge and two side edges extending between this base edge and this free end edge; at least one of said wall portions (12) is arranged on said base wall (10) such that a side edge (12a, 12b) of said wall portion (12) is at a distance from the center of the base wall (10) which is different from the distance of the other side edge (12b, 12a) of that wall portion (12) relative to this center.

2. Anchoring (1) base (2) according to claim 1, **characterized in that** said wall portions (12) are regularly distributed over the entire periphery of said base wall (10) and **in that** each wall portion (12) is disposed on this base wall (10) as mentioned above, that is to say in such a manner that one side edge (12a, 12b) of a wall portion (12) is located at a distance from the center of the base wall (10) which is different from the distance of the other side edge (12b, 12a) of that wall portion (12) relative to the center.

3. Anchoring (1) base (2) according to claim 1 or claim 2, **characterized in that** at least one side edge is serrated or crenellated.

4. Anchoring (1) base (2) according to claim 3, **characterized in that** the lateral edges of each wall portion (12) are serrated or crenellated.

5. Anchoring (1) base (2) according to one of claims 1 to 4, **characterized in that** the free end edge of each wall portion (12) has a pointed shape.

6. Anchoring (1) base (2) according to one of claims 1 to 5, **characterized in that** bone anchoring projections are arranged on the inner surface and / or on the outer surface of at least a portion wall (12).

7. Anchoring (1) base (2) according to one of claims 1 to 6, **characterized in that** it comprises a tubular wall (11) integral with said base wall (10) projecting from this wall base (10) on the same side thereof as said wall portions (12), said tubular wall (11) defining a cavity (13) for receiving with clamping a connection piece (7a to 7c) for connection to said articular part (3, 4).

8. Anchoring (1) base (2) according to claim 7, **characterized in that** said tubular wall (11) comprises bone anchoring projections on its external face, in particular in the form of collars.

9. Anchoring (1) base (2) according to one of claims 1 to 6, **characterized in that** it comprises a pyramidal central stud (35).

10. Anchoring (1) base (2) according to one of claims 1 to 6, **characterized in that** it comprises a central stud (36) forming a cavity (37) for receiving a graft and which wall is multi-perforated as to allow the growth of bone cells between the cancellous bone and this graft.

11. Set (1) of elements enabling the formation of a joint prosthesis (50), **characterized in that** it comprises:
- at least one anchoring base (2) according to one of claims 1 to 10;
- at least one articular part (3, 4) adapted to be mounted on this base (2),
- at least one resurfacing part (30 to 32), that is to say for at least partially reconstituting the native articular surface;
- connecting means (7a to 7c, 13, 18) for assembling one or the other of these articular parts (3, 4) and resurfacing parts (30 to 32) on each anchoring base (2).

12. Set of elements according to claim 11, **characterized in that** it comprises a plurality of anchoring bases (2) of different sizes and dimensions.

13. Set of elements according to claim 11 or claim 12, **characterized in that** it comprises at least one articular part (3) forming a convex articular surface for the formation of a so-called "anatomical" shoulder prosthesis (50) and at least one articular part (4) forming a concave articular surface for the formation of a so-called "reverse" shoulder prosthesis.

14. Set of elements according to one of claims 11 to 13, **characterized**:
- in that said connecting means comprise a conical cavity (13) on each anchoring base (2) and a conical cavity on at least one articular part (3) and connecting parts (7a to 7c) having double conical connecting portions, one of these conical connecting portions being capable to be engaged and retained in the conical cavity (13) of an anchoring base (2) and the other of these conical connecting portions being capable to be engaged and retained in the conical cavity (18) of the articular part (3); and
- in that the set (1) of elements comprises at least one straight connection part (7a), at least one angled connecting part (7b), that is to say, of which the axis of a connecting portion forms an angle different from 180° with the axis of the other connecting portion, and at least one offset connecting part (7c), that is to say, of which the axis of a connecting portion is shifted, at the connecting interface of this portion with the other portion, with respect to the axis of the other portion.
